# FASCICULE DE BREVET EUROPEEN

(11) **EP 0 399 899 B1**
(45) Date de publication et mention de la délivrance du brevet: **13.04.1994**
(21) Numéro de dépôt: 90401366.1
(22) Date de dépôt: 22.05.1990
(51) Int. Cl.: C07D 301/26, C07D 301/02, C07D 303/23, C07C 305/10

(54) **Procédé de préparation d'éthers aromatiques**
Verfahren zur Herstellung von aromatischen Ethern
Process for the preparation of aromatic ethers

(30) Priorité: 23.05.1989 FR 8906701
(43) Date de publication de la demande: 28.11.1990
(73) Titulaire: RHONE-POULENC SANTE, 92160 Antony (FR)
(72) Inventeur: Botannet, Bernard, F-38200 Vienne (FR); Le Fur, Isidore, F-94320 Thiais (FR); Massonneau, Viviane, F-69130 Ecully (FR); Mulhauser, Michel, F-69130 Ecully (FR)
(74) Mandataire: Pilard, Jacques

(56) Documents cités:
- DE-B- 1 815 808
- GB-A- 1 166 998
- US-A- 4 261 906
- JOURNAL OF THE AMERICAN CHEMICAL SOCIETY, vol. 110, no. 22, 26 octobre 1988,pages 7538-7539, American Chemical Society, Gaston, PA, US; Y. GAO et al.:"Vicinal diol cyclic sulfates: Like epoxides only more reactive"

## Description

La présente invention concerne un procédé de préparation d'éther aromatiques de formule générale : 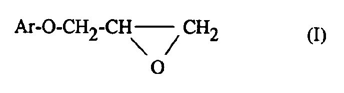
dans laquelle Ar représente un radical aromatique ou un radical aromatique hétérocyclique éventuellement substitué.

Les produits de formule générale (I) sont en particulier des intermédiaires pour la préparation de β-bloquants adrénergiques qui se caractérisent par la présence d'une chaîne alkylamino-3 hydroxy-2 propoxy, tels que l'acébutolol, l'aténolol, le propranolol, le métoprolol, le timolol, le nadolol ou le pindolol.

Généralement, les β-bloquants adrénergiques peuvent être obtenus, par exemple selon DE 1815808, par action d'une amine primaire sur l'époxyde de formule générale (I) qui est lui-même obtenu par action de l'épichlorhydrine sur un dérivé de formule générale :

Ar-OH (II)

dans laquelle Ar est défini comme précédemment. Cependant il est nécessaire d'utiliser un excès d'épichlorhydrine pour limiter la formation de produits secondaires tels que Ar-O-CH₂-CH(OH)-CH₂-O-Ar. Malgré l'excès d'épichlorhydrine, la formation de produits secondaires entraîne une diminution importante du rendement en produit de formule générale (I) et une purification nécessaire du produit de formule générale (I) ou des produits obtenus par la mise en oeuvre du produit de formule générale (I).

Y. Gao et K.B. Sharpless, J. Amer. Chem. Soc., 110, 7538-7539 (1988) enseignent que les sulfates de diols vicinaux peuvent constituer une alternative aux époxydes.

Il a maintenant été trouvé, et c'est ce qui fait l'objet de la présente invention, que les éthers aromatiques de formule générale (I) peuvent être obtenus, avec de bons rendements, sans formation de produits secondaires, par action d'un sulfate d'halogéno-3 propanediol-1,2 de formule générale : 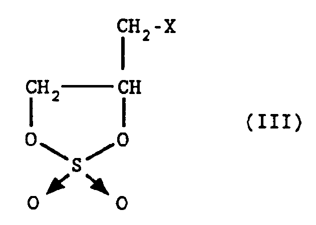
dans laquelle X représente un atome d'halogène (chlore, brome) sur un phénol de formule générale (II) dans laquelle Ar est défini comme précédemment en passant intermédiairement et successivement par les produits de formule générale : 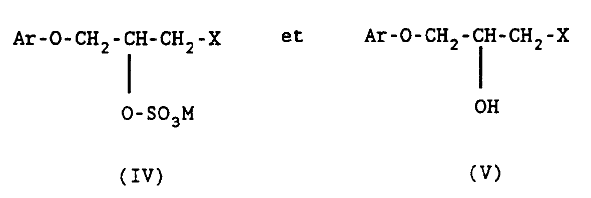
dans lesquelles Ar et X sont définis comme précédemment et M représente un atome d'hydrogène ou un atome de métal alcalin ou un ion ammonium, qu'il n'est pas nécessaire d'isoler.

Généralement la condensation du sulfate cyclique de formule générale (III) sur le dérivé de formule générale (II) est effectuée dans l'eau ou dans un solvant organique ou dans un milieu hydroorganique à une température comprise entre 0 et 80°C en présence d'une base choisie parmi les hydroxydes de métaux alcalins (soude), les carbonates ou bicarbonates de métaux alcalins ou alcalino-terreux (carbonate de sodium), l'ammoniaque ou les hydroxydes d'ammonium quaternaires (hydroxyde de tétrabutylammonium).

Comme solvants organiques peuvent être utilisés les nitriles (acétonitrile), les cétones (acétone), les alcools (éthanol), les esters (acétate d'éthyle), les amides (diméthylformamide) ou les hydrocarbures aliphatiques halogénés (chlorure de méthylène).

Généralement, on utilise un léger excès molaire, de préférence voisin de 10 %, de sulfate cyclique de formule générale (III) par rapport au phénol de formule générale (II).

Lorsque l'on utilise un phénol de formule générale (II) portant des substituants pouvant réagir avec le sulfate cyclique de formule générale (III), il peut être avantageux de les protéger par des groupements protecteurs convenables qui peuvent être éliminés ultérieurement sans toucher au reste de la molécule.

Le produit de formule générale (I) est isolé du mélange réactionnel par application des techniques habituelles.

Le sulfate d'halogéno-3 propanediol-1,2 de formule générale (III) peut être obtenu par oxydation du sulfite correspondant.

Généralement l'oxydation est réalisée au moyen d'un hypohalogénite (hypochlorite ou hypobromite alcalin ou alcalino-terreux), de préférence l'hypochlorite de sodium, de potassium ou de calcium, en présence d'une quantité catalytique d'un dérivé du ruthénium choisi de préférence parmi l'oxyde de ruthénium (IV) (RuO₂) et le chlorure de ruthénium (RuCl₃).

Le procédé peut être mis en oeuvre en milieu aqueux ou hydroorganique biphasique.

Lorsque le procédé est mis en oeuvre en milieu hydroorganique biphasique, le solvant est généralement choisi parmi les hydrocarbures aliphatiques ou cycloaliphatiques éventuellement halogénés tels que l'hexane, le cyclohexane, le chlorure de méthylène, le chloroforme, le tétachlorure de carbone ou le dichloroéthane, et les esters tels que l'acétate de méthyle ou l'acétate d'éthyle.

Généralement on utilise une quantité catalytique de dérivé du ruthénium comprise entre 10⁻⁶ et 10⁻¹ mole par mole de sulfite cyclique.

Le sulfate cyclique de formule générale (III) est séparé du mélange réactionnel selon les techniques habituelles.

Le sulfite cyclique d'halogéno-3 propanediol-1,2 peut être préparé selon les méthodes connues et en particulier selon le procédé décrit par D.S. Breslow et H. Solnik, "The Chemistry of Heterocyclic Compounds - Multi-Sulphur and Sulphur and Oxygen 5- and 6- Membered Heterocycles", 1966, Part 1, p. 1 et Part II, p. 663 ou par H.F. Van Woerden, Chem. Rev., 63, 557 (1963).

Les β-bloquants adrénergiques contenant une chaîne alkylamino-3 hydroxy-2 propoxy peuvent être obtenus selon les méthodes connues par action d'une amine primaire sur le produit de formule générale (I).

Les β-bloquants adrénergiques contenant une chaîne alkylamino-3 hydroxy-2 propoxy peuvent aussi être obtenus par action d'une amine primaire sur le produit de formule générale (IV) suivi du remplacement du reste sulfate (SO₄H) par un radical hydroxy en milieu acide ou basique.

Le produit de formule générale (IV), qui est un produit nouveau constituant un autre objet de la présente invention, peut être obtenu en contrôlant la réaction de condensation du sulfate cyclique de formule générale (III) sur le dérivé de formule générale (II). Lorsque tout le sulfate cyclique est consommé, le mélange réactionnel est acidifié et le produit de formule générale (IV) est isolé.

L'exemple suivant illustre la présente invention.

### EXEMPLE

Dans un réacteur muni d'une agitation, on introduit 2,21 g d'acétyl-2 butyramido-4 phénol (10 mmoles), 2 g de sulfate de chloro-3 propanediol-1,2 (11,5 mmoles) et 30 cm3 d'acétonitrile. On ajoute ensuite 0,5 g de soude (15 mmoles) en solution dans 2,5 cm3 d'eau.

On agite pendant 5 heures à une température voisine de 20°C jusqu'à obtenir un mélange homogène. On chauffe ensuite pendant 25 minutes à 50°C. Le précipité qui apparaît est séparé par filtration. On ajoute alors 0,9 g de soude en solution dans 1,5 cm3 d'eau. Après 10 minutes d'agitation, le précipité formé est séparé par filtration. La solution obtenue est concentrée sous pression réduite. On obtient ainsi 2,5 g d'(acétyl-2 butyramido-4 phénoxy)-1 époxy-2,3 propane dont les caractéristiques sont identiques à celles d'un échantillon authentique.

Le rendement est voisin de 90 %.

Le sulfate de chloro-3 propanediol-1,2 peut être préparé de la manière suivante :

Dans un ballon de 250 cm3, on introduit 4,96 g de sulfite de chloro-3 propanediol-1,2 (0,003 mole) et 25 cm3 d'eau. Après refroidissement à 0°C, on ajoute, sous agitation, 22,5 cm3 d'une solution aqueuse d'hypochlorite de sodium à 2 moles par litre (soit 0,045 mole) contenant 4,5 mg d'oxyde de ruthénium (IV) dihydrate (0,027 x 10-3 mole).

On poursuit l'agitation pendant 5 minutes à 2°C. On extrait le mélange réactionnel par 2 fois 20 cm3 de dichlorométhane. La phase organique est traitée par 0,5 cm3 d'isopropanol puis est lavée par 15 cm3 d'eau. Après séchage de la phase organique et concentration sous pression réduite (2 mm de mercure ; 0,47 kPa), on obtient 3,95 g de sulfate de chloro-3 propanediol-1,2 sous forme d'un liquide incolore.

Le rendement est de 76,4 %.

## Revendications

1. Procédé de préparation d'éthers aromatiques de formule générale : 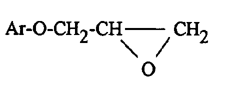 dans laquelle Ar représente un radical aromatique ou un radical aromatique hétérocyclique éventuellement substitué caractérisé en ce que l'on fait réagir un sulfate d'halogéno-3 propanediol-1,2 de formule générale : 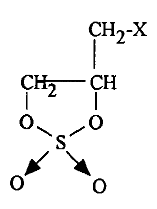 dans laquelle X représente un atome d'halogène choisi parmi le chlore ou le brome, sur un produit de formule générale :
Ar-OH
dans laquelle Ar est défini comme précédemment en passant intermédiairement par les produits de formule générale : 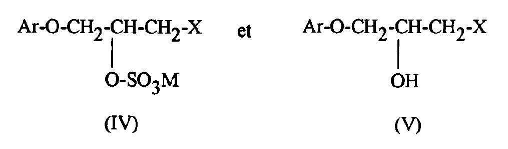 dans lesquelles Ar et X sont définis comme précédemment, et M représente un atome d'hydrogène ou un atome de métal alcalin ou un ion ammonium, qu'il n'est pas nécessaire d'isoler, en opérant dans l'eau ou dans un solvant organique ou dans un milieu hydroorganique en présence d'une base.

2. Procédé selon la revendication 1 caractérisé en ce que le solvant organique est choisi parmi les nitriles, les cétones, les alcools, les esters, les amides et les hydrocarbures aliphatiques halogénés.

3. Procédé selon l'une des revendications 1 ou 2 caractérisé en ce que la base est choisie parmi les hydroxydes ou carbonates ou bicarbonates de métaux alcalins ou alcalino-terreux, l'ammoniaque et les hydroxydes d'ammonium quaternaires.

4. Procédé selon l'une des revendications 1 à 3 caractérisé en ce que l'on opère à une température comprise entre 0 et 80°C.

5. Le produit de formule générale : 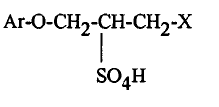 dans laquelle Ar représente un radical aromatique ou un radical aromatique hétérocyclique et X représente un atome d'halogène choisi parmi le chlore et le brome, éventuellement sous forme d'un sel de métal alcalin ou d'ammonium.

6. Utilisation d'un produit selon la revendication 5 pour la préparation de β-bloquants adrénergiques portant une chaîne alkylamino-3 hydroxy-2 propoxy caractérisé en ce que l'on fait réagir une amine primaire sur le produit selon la revendication 5 puis l'on remplace le reste sulfate par un radical hydroxy en milieu acide ou basique.

## Patentansprüche

1. Verfahren zur Herstellung von aromatischen Ethern der allgemeinen Formel 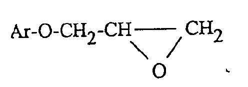 worin Ar für einen aromatischen Rest oder einen heterocyclischen aromatischen Rest, der gegebenenfalls substituiert ist, steht, dadurch gekennzeichnet, daß man ein 3-Halogen-l,2-propandiolsulfat der allgemeinen Formel 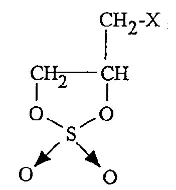 worin x ein Halogenatom, ausgewählt unter Chlor oder Brom, bedeutet, mit einem Produkt der allgemeinen Formel
Ar-OH
worin Ar wie vorstehend definiert ist, umsetzt, wobei man intermediär über die Produkte der allgemeinen Formel 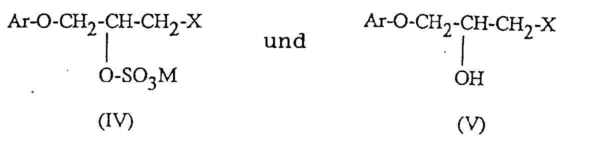 worin Ar und X wie vorstehend definiert sind und M ein Wasserstoffatom oder ein Alkalimetallatom oder ein Ammoniumion bedeutet, gelangt, die nicht isoliert werden müssen, wobei man in Wasser oder in einem organischen Lösungsmittel oder in einem wäßrig-organischen Milieu in Gegenwart einer Base arbeitet.

2. Verfahren gemäß Anspruch 1, dadurch gekennzeichnet, daß das organische Lösungsmittel unter den Nitrilen, den Ketonen, den Alkoholen, den Estern, den Amiden und den halogenierten aliphatischen Kohlenwasserstoffen ausgewählt wird.

3. Verfahren gemäß einem der Ansprüche 1 oder 2, dadurch gekennzeichnet, daß die Base unter den Alkalimetall- oder Erdalkalimetallhydroxiden oder -carbonaten oder -bicarbonaten, Ammoniak und den quaternären Ammoniumhydroxiden ausgewählt wird.

4. Verfahren gemäß einem der Ansprüche 1 bis 3, dadurch gekennzeichnet, daß man bei einer Temperatur zwischen 0 und 80°C arbeitet.

5. Produkt der allgemeinen Formel 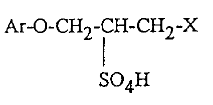 worin Ar für einen aromatischen Rest oder einen heterocyclischen aromatischen Rest steht und X ein Halogenatom, ausgewählt unter Chlor und Brom, bedeutet, gegebenenfalls in Form eines Alkalimetall- oder Ammoniumsalzes.

6. Verwendung eines Produkts gemäß Anspruch 5 zur Herstellung von adrenergischen β-Blockern, die eine 3-Alkylamino-2-hydroxypropoxykette enthalten, dadurch gekennzeichnet, daß man ein primäres Amin mit dem Produkt gemäß Anspruch 5 umsetzt, wonach man den Sulfatrest durch eine Hydroxygruppe in saurem oder basischem Milieu ersetzt.

## Claims

1. Process for the preparation of aromatic ethers of general formula: 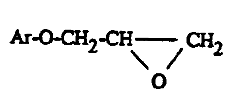 in which Ar denotes an aromatic radical or a heterocyclic aromatic radical, optionally substituted, in which a 3-halo-1,2-propanediol sulphate of general formula: 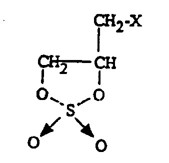 in which X denotes a halogen atom chosen from chlorine and bromine, is reacted with a product of general formula:
Ar-OH
in which Ar is defined as above, passing intermediately via the products of general formula: 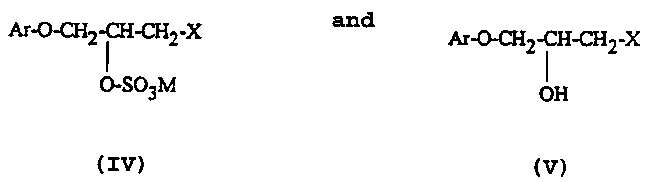 in which Ar and X are defined as above and M denotes a hydrogen atom or an alkali metal atom or an ammonium ion, which it is unnecessary to isolate, the operation being carried out in water or in an organic solvent or in a hydroorganic medium in the presence of a base.

2. Process according to Claim 1, characterized in that the organic solvent is chosen from nitriles, ketones, alcohols, esters, amides and halogenated aliphatic hydrocarbons.

3. Process according to either of Claims 1 and 2, characterized in that the base is chosen from alkali or alkaline-earth metal hydroxides or carbonates or bicarbonates, aqueous ammonia and quaternary ammonium hydroxides.

4. Process according to one of Claims 1 to 3, characterized in that it is carried out at a temperature of between 0 and 80°C.

5. The product of general formula: 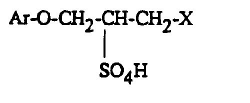 in which Ar denotes an aromatic radical or a heterocyclic aromatic radical and X denotes a halogen atom chosen from chlorine and bromine, optionally in the form of an alkali metal or ammonium salt.

6. Use of the product according to Claim 5 for the preparation of adrenergic β-blockers bearing a 3-alkylamino-2-hydroxypropoxy chain, characterized in that a primary amine is reacted with the product according to Claim 5 and the sulphate residue is then replaced by a hydroxyl radical in acidic or basic medium.
